# EUROPEAN PATENT APPLICATION

(11) **EP 2 500 380 A1**
(43) Date of publication of application: **19.09.2012**
(21) Application number: 10829873.8
(22) Date of filing: 04.11.2010
(51) Int. Cl.: C08L 23/10, A61L 27/00, A61L 29/00, A61L 31/00, C08L 53/00

(54) **MEDICAL OR BIOCHEMICAL RESIN COMPOSITION AND RESIN MOLDED PRODUCT**

(30) Priority: 13.11.2009 JP 2009260387
(71) Applicant: Richell Corporation, Toyama-shi Toyama 9390592 (JP)
(72) Inventor: YAMASHITA Kazuyuki, Toyama-shi Toyama 939-0592 (JP); SHIROKI Masahiro, Toyama-shi Toyama 939-0592 (JP)
(74) Representative: Winter, Brandl, Fürniss, Hübner, Röss, Kaiser, Polte - Partnerschaft
(86) International application number: PCT/JP2010/069557
(87) International publication number: WO 2011/058914

(57) **Abstract**

A resin composition that exhibits excellent moldability and excellent mechanical strength, and may suitably be used to produce a resin product that suppresses elution from the inner wall thereof is disclosed. In particular, a medical or biochemical resin composition that prevents adsorption of proteins, polypeptides, and peptides, and exhibits biocompatibility, antithrombogenicity, elution resistance, and moldability (e.g., injection moldability), and a resin molded product are disclosed. The medical or biochemical resin composition includes a polypropylene-based resin, and a hydrogenated derivative of a block copolymer shown by X-Y (wherein X represents s a polymer block immiscible with the polypropylene-based resin, and Y represents an elastomer polymer block of a conjugated diene), the polypropylene-based resin having a content of an antioxidant of 1 mass% or less and a content of an additive of 0.5 mass% or less, the additive being used to disperse the antioxidant in the polypropylene-based resin.

## Description

### TECHNICAL FIELD

The present invention relates to a resin composition that exhibits excellent moldability and excellent mechanical strength, and may suitably be used to produce a resin product that suppresses elution from the inner wall thereof. In particular, the invention relates to a medical or biochemical resin composition that exhibits an excellent capability to prevent adsorption of proteins, polypeptides, peptides, and the like, and exhibits excellent biocompatibility, antithrombogenicity, and the like, and a resin molded product.

### BACKGROUND ART

In recent years, a proteomic analysis technique that analyzes the proteome present in a biological system has been developed. For example, mass spectrometry that utilizes matrix-assisted laser desorption ionization (MALDI) can be implemented using a small amount of sample.

MALDI utilizes a mixed solvent of water and acetonitrile or the like (hereinafter referred to as "organic mixed solvent") during a sample preparation process that subjects sample proteins to enzymatic digestion to obtain peptide fragments.

Therefore, a container or a tube used for MALDI is required to suppress adsorption of proteins, polypeptides, or peptides, and elution into an organic solvent or an organic mixed solvent, which would affect the analysis.

Such a container or a tube is also required to exhibit mechanical strength, moldability, and solvent resistance necessary for a medical product.

A medical product (e.g., artificial organ, catheter, stent, medical tube, blood circuit, blood storage container, permeable membrane, or glass syringe) may be used to inject or administer a liquid preparation such as a polypeptide hormone. In this case, it is necessary to prevent adsorption of the preparation on the surface of the medical product.

Such a medical product is also required to exhibit compatibility with blood and tissue.

A polyester, a polysulfone, polyvinyl chloride, polystyrene, a silicone resin, a polyolefin, a polymethacrylate, a fluorine-containing resin, and the like have been known as a polymer material used for proteomic analysis products and medical products utilized in the field of biochemistry.

These polymer materials have satisfactory mechanical strength. However, these polymer materials have an insufficient capability to prevent adsorption of proteins and the like, and have insufficient biocompatibility and antithrombogenicity due to a hydrophobic surface.

A container made of a polypropylene-based resin has been widely used as proteomic analysis products and medical products.

The polypropylene-based resin is classified into crystalline homopolypropylene, block polypropylene, and random polypropylene. These polypropylene-based resins contain additives such as an antioxidant, a UV inhibitor, an antistatic agent, a nucleating agent, and a lubricant (slipping agent) taking account of moldability, product lifetime, transparency, and the like.

A container made of a polypropylene-based resin can be conveniently used in experiments that utilize an aqueous solvent. However, additives contained in the polypropylene-based resin may be eluted from the inner wall of the container when using an organic mixed solvent.

Since such an eluate may hinder LC UV detection and mass spectrometry during proteomic analysis, it is desirable to use a tube (container) that does not produce an eluate as much as possible.

In the field of biochemistry, segmented polyurethane is well known as a material that improves biocompatibility and antithrombogenicity (see Patent Document 1).

The segmented polyurethane suppresses adhesion of platelets due to a microphase-separated structure of a rigid aromatic urethane bond site and a flexible polyether bond site. However, the segmented polyurethane also produces an eluate, for example.

A material may be provided with antithrombogenicity by utilizing a 2-hydroxyethyl methacrylate (HEMA)-styrene-HEMA block copolymer or the like so that the surface of the polymer has a microphase-separated structure of a hydrophilic site and a hydrophobic site (see Non-patent Document 1).

However, such a material exhibits insufficient moldability and the like in practice.

A material may also be provided with antithrombogenicity by utilizing a poly(alkyl aryl ether)sulfone copolymer having a solubility parameter within a given range so that the surface of the polymer has a microphase-separated structure of a hydrophilic site and a hydrophobic site (see Patent Document 2).
Patent Document 1: JP-A-60-238315
Patent Document 2: JP-A-2000-38488

Non-patent Document 1: "Trans. ASAIO", Vol. 33, Section 596 (1987)

### SUMMARY OF THE INVENTION

### TECHNICAL PROBLEM

An object of the invention is to provide a resin composition that exhibits an excellent capability to prevent adsorption of proteins and the like, exhibits excellent biocompatibility and antithrombogenicity, produces only a small amount of eluate when using an organic solvent or an organic mixed solvent, exhibits excellent moldability (e.g., injection moldability), and exhibits mechanical strength and solvent resistance when used as a medical or biochemical product, and a medical or biochemical resin molded product using the resin composition.

### SOLUTION TO PROBLEM

A medical or biochemical resin composition according to one aspect of the invention includes a polypropylene-based resin, and a hydrogenated derivative of a block copolymer shown by X-Y, the polypropylene-based resin having a content of an antioxidant of 1 mass% or less and a content of an additive of 0.5 mass% or less, the additive being used to disperse the antioxidant in the polypropylene-based resin (the unit "mass%" is hereinafter referred to as "%").

The antioxidant is mainly used to suppress decomposition of the amorphous part of the polypropylene-based resin. Since the resin composition is obtained by mixing the polypropylene-based resin with the hydrogenated derivative, it is preferable that the resin composition include the polypropylene-based resin and the hydrogenated derivative of the block copolymer shown by X-Y, have a content of an antioxidant of 1 mass% or less, and have a content of an additional additive of 0.5 mass% or less in order to suppress the effects of the additive included in the hydrogenated derivative.

The polymer block X represents a polymer block immiscible with the polypropylene-based resin, and the polymer block Y represents an elastomer polymer block of a conjugated diene.

When the polymer block X is immiscible with the polypropylene-based resin, and the polymer block Y is miscible with the polypropylene-based resin, the polymer block X forms a microdomain having a size approximately equal to the radius of gyration, so that adsorption of proteins, polypeptides, and peptides is suppressed.

Proteins typically have a molecular weight of 75,000 to 200,000. For example, bovine serum albumin (BSA) has a molecular weight of about 67,000.

The size of the protein molecule differs depending on the three-dimensional structure. The size of the protein molecule is normally 100 to 500 nm in an extended state, and is normally 4 to 8 nm in a folded state.

A polypeptide has a structure in which ten or more amino acids are bonded via a peptide bond. An oligopeptide has a molecular weight of about 1000 to 3000 and an average particle size of about 2.5 nm.

A peptide has a structure in which two or more and less than ten amino acids are bonded via a peptide bond. A peptide is present in the shape of a chain, coil, ball, or the like. It is considered that a peptide may have a size of 2.5 nm or less.

Therefore, it is preferable to form a microdomain taking account of the above molecular size.

In a field of plastic resin, an antistatic agent that prevents occurrence of static electricity during molding, processing, usage, or the like, a slipping agent (lubricant), a plasticizer, or a release agent that improves moldability, an antioxidant that prevents a thermal deterioration and an oxidative deterioration, a nucleating agent that improves transparency, and the like are used to be added to a synthetic resin.

The inventors of the invention found that such an additive may affect the capability to prevent adsorption of proteins or the like, or may be eluted to affect proteomic analysis, and also found that the content of an antioxidant in the polypropylene-based resin should be 1 mass% or less, the content of an additive used to disperse the antioxidant in the polypropylene-based resin should be 0.5 mass% or less, and an additional additive should not be included in the polypropylene-based resin.

Specifically, it is indispensable to add an antioxidant to the polypropylene-based resin in order to prevent oxidation of the amorphous part, and a neutralizer may be added in order to improve the dispersibility of the antioxidant in the resin and neutralize a polymerization catalyst. However, it is preferable not to add an antistatic agent, a plasticizer, a release agent, and the like.

It is preferable to add an additive in an amount as small as possible. The content of the neutralizer is preferably 0.5% or less, and more preferably 0.3% or less. It is preferable not to add an antistatic agent, a plasticizer, a release agent, a nucleating agent, and the like.

It is preferable to limit the total content of additives other than the antioxidant in the resin composition to 0.5% or less.

Examples of the antioxidant include phenol-type antioxidants, phosphite-type antioxidants, and sulfur-type antioxidants. Any of these antioxidants may be used.

Examples of hindered phenol-type antioxidants include triethylene glycol bis[3-(3-t-butyl-5-methyl-4-hydroxyphenyl)propionate],
pentaerythrityltetrakis[3-(3,5-di-t-butyl-4-hydroxyphenyl)propionate], octadecyl-3-(3,5-di-t-butyl-4-hydroxyphenyl)propionate,
1,3,5-trimethyl-2,4,6-tris(3,5-di-t-butyl-4-hydroxybenzyl)benzene,
3,9-bis[2- {3-(3-t-butyl-4-hydroxy-5-methylphenyl)propionyloxy} -1,1-dimethylethyl]-2,4,8,10-tetraoxaspiro[5,5]undecane, and the like.

Examples of phosphorus-type antioxidants include tris(2,4-di-t-butylphenyl) phosphite, tetrakis(2,4-di-t-butylphenyl)-4,4'-biphenylene phosphite, tris(nonylphenyl) phosphite, bis(2,4-di-t-butylphenyl)pentaerythritol diphosphite, distearylpentaerythritol diphosphite, and the like.

Examples of thioether-type antioxidants include dilauryl 3,3'-thiodipropionate, dimyristyl 3,3'-thiodipropionate, distearyl 3,3'-thiodipropionate, pentaerythrityl-tetrakis(3-laurylthiopropionate), ditridecyl 3,3'-thiodipropionate, and the like.

As the polypropylene-based resin, a polypropylene homopolymer or a polypropylene random copolymer that includes an α-olefin such as ethylene, butene-1, or hexene-1 may be used.

Among these, a polypropylene homopolymer (homopolypropylene resin) is preferable.

A Natta catalyst, a ferrocene catalyst, or the like may be used as a polypropylene polymerization catalyst.

Examples of the polymer block X of the hydrogenated derivative include a polymer produced by polymerizing a vinyl aromatic monomer (e.g., styrene), ethylene, a methacrylate (e.g. methyl methacrylate), or the like.

The hydrogenated derivative of the block copolymer shown by X-Y may be a copolymer shown by (X-Y)ₙ (n=1 to 5), X-Y-X, Y-X-Y, or the like.

Examples of the polymer block X of the hydrogenated derivative include a polystyrene polymer block and a polyolefin polymer block. Examples of the polystyrene polymer block include a polymer block that includes one or more aromatic vinyl compounds selected from styrene, α-methylstyrene, o-methylstyrene, m-methylstyrene, p-methylstyrene, 2,4-dimethylstyrene, vinylnaphthalene, and vinylanthracene as a monomer unit.

Examples of the polyolefin polymer block include copolymers of ethylene and an α-olefin having 3 to 10 carbon atoms.

A nonconjugated diene may be polymerized in the polymer block.

Examples of the olefin include propylene, 1-butene, 3-methyl-1-butene, 1-pentene, 4-methyl-1-pentene, 1-hexene, 1-pentene, 1-octene, 1-decene, and the like.

Examples of the nonconjugated diene include 1,4-hexadiene, 5-methyl-1,5-hexadiene, 1,4-octadiene, cyclohexadiene, cyclooctadiene, cyclopentadiene, 5-ethylidene-2-norbornene, 5-butylidene-2-norbornene, 2-isopropenyl-5-norbomene, and the like.

Specific examples of the copolymer include an ethylene-propylene copolymer, an ethylene-1-butene copolymer, an ethylene-1-octene copolymer, an ethylene-propylene-1,4-hexadiene copolymer, an ethylene-propylene-5-ethylidene-2-norbornene copolymer, and the like.

Examples of the polymer block Y before hydrogenation include polybutadiene that includes at least one group selected from the group consisting of a 2-butene-1,4-diyl group and a vinylethylene group as a monomer unit, and polyisoprene that includes at least one group selected from the group consisting of a 2-methyl-2-butene-1,4-diyl group, an isopropenylethylene group, and a 1-methyl-1-vinylethylene group as a monomer unit.

Example of the polymer block Y before hydrogenation also include an isoprene-butadiene copolymer that includes an isoprene unit and a butadiene unit as the main monomer units, the isoprene unit being at least one group selected from the group consisting of a 2-methyl-2-butene-1,4-diyl group, an isopropenylethylene group, and a 1-methyl-1-vinylethylene group, and the butadiene unit being a 2-butene-1,4-diyl group and/or a vinylethylene group.

The arrangement of the butadiene unit and the isoprene unit may be a random arrangement, a block arrangement, or a tapered block arrangement.

Further examples of the polymer block Y before hydrogenation include a aromatic vinyl compound-butadiene copolymer that includes an aromatic vinyl compound unit and a butadiene unit as the main monomer units, the aromatic vinyl compound unit being at least one monomer unit selected from styrene, α-methylstyrene, o-methylstyrene, m-methylstyrene, p-methylstyrene, 2,4-dimethylstyrene, vinylnaphthalene, and vinylanthracene, and the butadiene unit being a 2-butene-1,4-diyl group and/or a vinylethylene group.

The arrangement of the aromatic vinyl compound unit and the butadiene unit may be a random arrangement, a block arrangement, or a tapered block arrangement.

The hydrogenation state of the polymer block Y may be partial hydrogenation or complete hydrogenation. Note that it is preferable that 50% or more, and more preferably 80% or more of the carbon-carbon double bonds of the butadiene unit and/or the isoprene unit be hydrogenated in order to improve mutual solubility, heat resistance (thermal deterioration resistance), or weatherability.

The raw materials for the resin composition are easily available if the polymer block X of the hydrogenated derivative is polystyrene, and the polymer block Y before hydrogenation is 1,2-polyisoprene, 3,4-polyisoprene, and/or 1,4-polyisoprene.

Since the styrene component has low miscibility with the polypropylene-based resin or the like, it takes time to mix the styrene component with polypropylene as the ratio of the styrene component increases. Therefore, when using a hydrogenated derivative containing a large amount of styrene component, it is preferable to sufficiently mix the styrene component and polypropylene in advance to prepare a masterbatch.

The raw materials for the resin composition are also easily available if the polymer block X of the hydrogenated derivative is polystyrene, and the polymer block Y before hydrogenation is 1,2-polybutadiene and/or 1,4-polybutadiene.

The details of the term "miscibility" are described below.

When the polymer block X is immiscible with the polypropylene-based resin, the polymer block X forms a microdomain having a size approximately equal to the radius of gyration. The microdomain may be confirmed by observation using an atomic force microscope or a transmission electron microscope, or measurement and analysis of the scattering pattern of isolated domains using a small-angle X-ray scattering technique.

The glass transition temperature of the polymer block X changes to only a small extent when mixed with the polypropylene-based resin. This may be confirmed by differential scanning calorimetry (DSC), dynamic viscoelastic measurement, or the like.

When the polymer block Y is miscible with the polypropylene-based resin, the glass transition temperature of the polymer block Y and the glass transition temperature of the polypropylene-based resin change, and a new glass transition temperature is observed between the glass transition temperature of the polymer block Y and the glass transition temperature of the polypropylene-based resin.

Such a change in glass transition temperature may be confirmed by dynamic viscoelastic measurement or the like.

When the polymer blocks X and Y of the X-Y block copolymer are immiscible with the polypropylene-based resin, the X-Y block copolymer phase (that forms a microdomain structure formed of the phase of the polymer block X and the phase of the polymer block Y) and the polypropylene-based resin phase are separated. When the polymer block Y is miscible with the polypropylene-based resin, the space (interval) between the microdomains of the polymer block X increases, or the microdomains of the polymer block X are uniformly dispersed in the polypropylene-based resin.

Such a morphological change that occurs when the polymer block Y is miscible with the polypropylene-based resin may be confirmed by observing the relative positions of the microdomains using an atomic force microscope or a transmission electron microscope, or analyzing the distance between the microdomains using a small-angle X-ray scattering technique.

Since the medical or biochemical resin composition according to one aspect of the invention exhibits excellent moldability, the effective surface of a molded product produced by injection-molding the medical or biochemical resin composition has a root-mean-square surface roughness of 10 nm or less. The medical or biochemical resin composition according to one aspect of the invention also exhibits high mechanical strength, and has a tensile modulus of 100 MPa or more.

The term "effective surface" used herein refers to a surface that comes in contact with a reagent or the like during analysis or the like.

Since the mechanical strength of the molded product may decrease when the content of the hydrogenated derivative is too high, the content of the hydrogenated derivative in the resin composition is preferably 10 to 60%.

### ADVANTAGEOUS EFFECTS OF THE INVENTION

Since the medical or biochemical resin composition according to one aspect of the invention includes the polypropylene-based resin and the hydrogenated derivative of the block copolymer shown by X-Y, the polymer block X is a polymer block immiscible with the polypropylene-based resin, and the polymer block Y is a conjugated diene elastomer polymer block, a microdomain having a size approximately equal to the radius of gyration of the polymer block X is formed, so that adsorption of proteins, polypeptides, and peptides is suppressed.

Since the medical or biochemical resin composition includes an antioxidant and an optional neutralizer, but substantially does not include an additional additive, the amount of eluate is small when using an organic mixed solvent during analysis.

Since the polymer block Y is miscible with the polypropylene-based resin, the melting point of the resin composition decreases to only a small extent. Therefore, it is possible to provide mechanical strength and solvent resistance required for medical products and biochemical products.

### BRIEF DESCRIPTION OF DRAWINGS

FIG. 1 shows digested peptide fragment recovery results (in-gel digestion of BSA).
FIG. 2A and 2B shows an HPLC analysis chart (simple elution test, 50 ng concentrated sample and dried sample) when using sample containers RA, RB, RC, and W.
FIG. 3A and 3B shows an HPLC analysis chart (simple elution test, 20 ng concentrated sample and dried sample) when using sample containers RA, RB, RC, and W.
FIG. 4 shows HPLC analysis conditions.
FIG 5A and 5B shows an HPLC eluate analysis chart after proteomic analysis (50 ng concentrated sample and dried sample) when using a sample container RD.
FIG. 6A and 6B shows an HPLC eluate analysis chart after proteomic analysis (10 ng concentrated sample and dried sample) when using a sample container RD.
FIG. 7 shows the properties of a homopolypropylene resin "MA04A".
FIG. 8 shows a resin compositional ratio of a sample container subjected to evaluation.
FIG. 9 shows an example of the shape of sample container.
FIG. 10 shows the properties of a hydrogenated derivative "7311 ".
FIG. 11 shows a graph of the melting point and the heat of fusion of homopolypropylene and a hydrogenated derivative depending on the mixing ratio.
FIG. 12 shows a graph of the tensile modulus of homopolypropylene and a hydrogenated derivative depending on the mixing ratio.
FIG. 13 shows an atomic force micrograph.
FIG. 14A and 14B shows measurement results for root-mean-square roughness Rq.
FIG. 15 shows peptide adsorption test conditions.
FIG. 16 shows peptide adsorption test conditions.
FIG. 17 shows peptide adsorption test results.
FIG. 18 shows peptide adsorption test results.
FIG. 19 shows the Mascot score of each fraction.
FIG. 20 shows the Mascot score of each fraction.
FIG. 21 shows elution test conditions.
FIG. 22 shows elution test results.
FIG. 23 shows elution test results.
FIG. 24 shows elution test results.
FIG. 25 shows liquid removal test conditions.
FIG. 26 shows appearance after a liquid removal test.
FIG. 27 shows liquid residual rate measurement results.
FIG. 28 shows liquid residual rate measurement results.

### DESCRIPTION OF EMBODIMENTS

A proteomic analysis sample container was produced using a medical or biochemical resin composition according to one embodiment of the invention, and the peptide adsorption prevention capability and the elution resistance were evaluated.

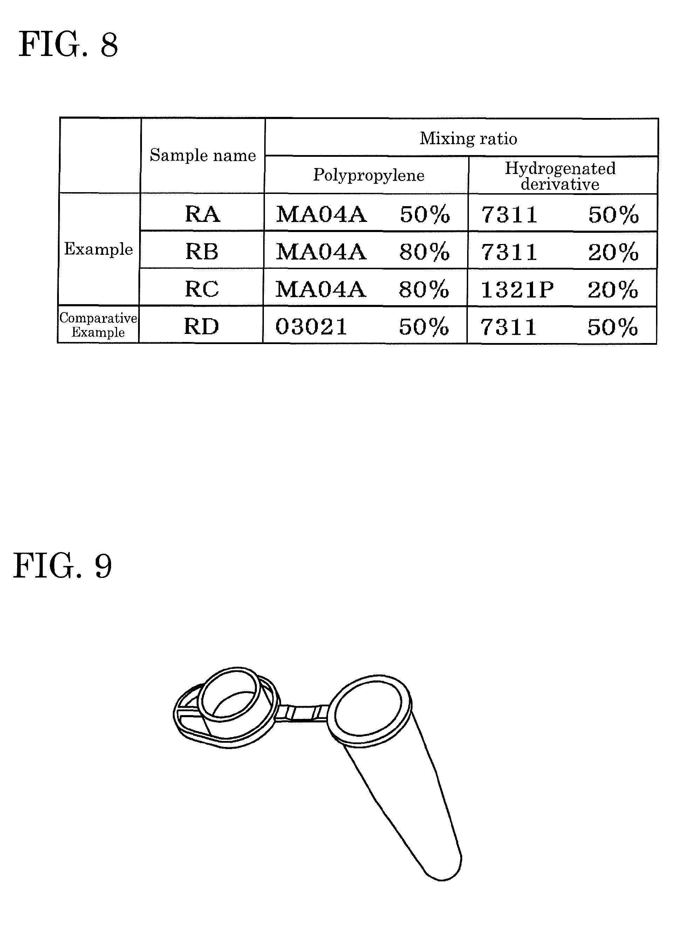

A sample container (capacity: 0.5 ml) shown in FIG. 9 was injection-molded using a resin composition including a polypropylene-based resin and a hydrogenated derivative in the ratio shown in FIG. 8, and subjected to proteomic analysis. Recovery of digested peptide fragments (in-gel digestion of BSA) was evaluated, and the amount of eluate was analyzed by HPLC.

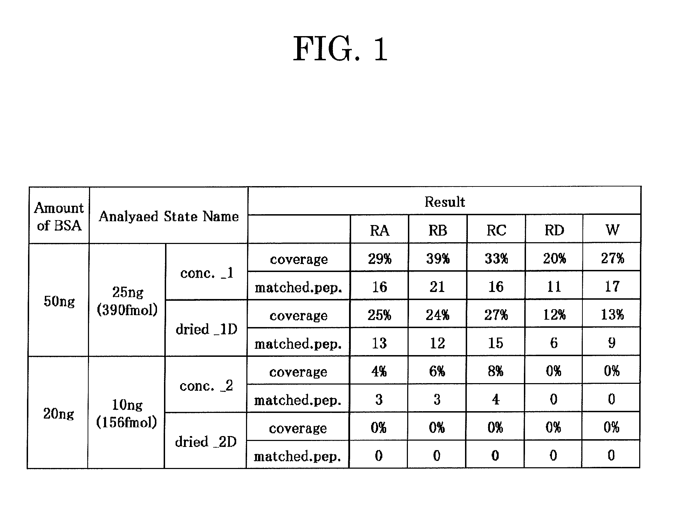

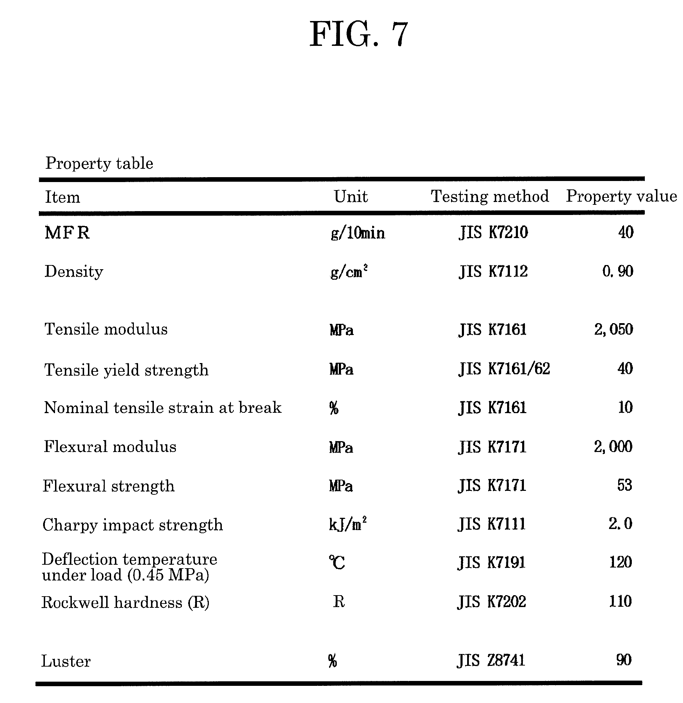

In FIG. 8, "MA04A" (used in the example) indicates a homopolypropylene resin "Novatec-PP MA04A" (manufactured by Japan Polypropylene Corporation) (indicated by "RA", "RB", or "RC" in FIG. 1 depending on the mixing ratio). FIG. 7 shows the property values of the homopolypropylene resin "MA04A".

The CAS number of the homopolypropylene resin "MA04A" is 9003-07-0. The homopolypropylene resin "MA04A" contains 0.05% of tetrakis[methylene-3-(3,5-di-t-butyl-4-hydroxyphenyl)propionate]methane (antioxidant), 0.05% of tris(2,4-di-t-butylphenyl) phosphite (antioxidant), 0.03% of magnesium aluminum hydroxide carbonate hydrate (neutralizer), and 0.10% of sodium 2,2-methylenebis(2,6-di-t-butylphenyl)phosphate (neutralizer), and does not contain an additional additive.

The hydrogenated derivative "7311" is a hydrogenated polystyrene-vinylpolyisoprene-polystyrene block copolymer "Hybrar 7311 S" (manufactured by Kuraray Co., Ltd.) (styrene content: 12 wt%, the vinylpolyisoprene phase is hydrogenated). The hydrogenated derivative "7311" is a thermoplastic polymer that exhibits rubber elasticity. The CAS number of the hydrogenated derivative "7311" is 132778-07-5. The hydrogenated derivative "7311" contains an antioxidant (1% or less) and an anti-blocking agent (1% or less).

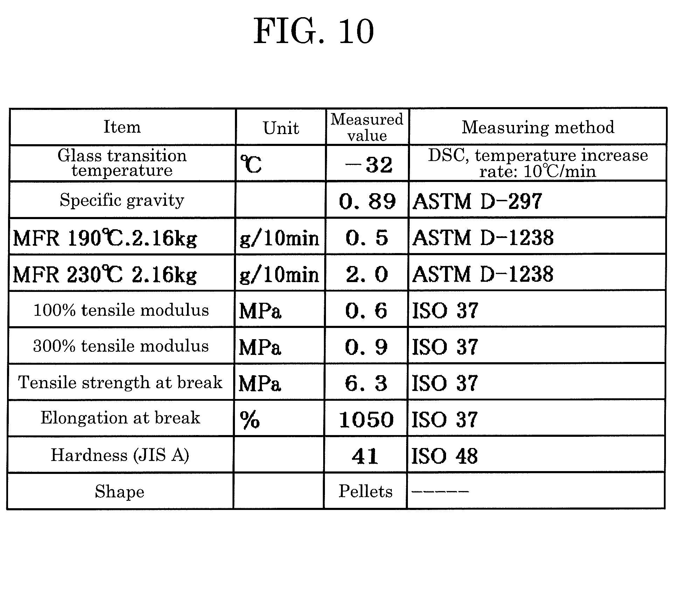

The hydrogenated derivative "7311" has excellent affinity for a polyolefin and a styrene polymer. FIG. 10 shows the property values of the hydrogenated derivative "7311 ".

The hydrogenated derivative "1321P" is a hydrogenated polystyrene-butadiene elastomer "Dynaron 1321P" (manufactured by JSR Corporation) (styrene content: 10%).

The polypropylene resin "03021" (used in the comparative example) is a random polypropylene resin "J-3021GR" (manufactured by Idemitsu Kosan Co., Ltd.) (injection molding grade) (indicated by "RD" in FIG. 1).

The polypropylene resin "03021" has an MFR of 33 g/10 min, a density of 0.9 g/cm³, a tensile modulus of 1000 MPa, a flexural modulus of 1000 MPa, and a Rockwell hardness of 76R, and contains an antistatic agent and the like (content: >0.5%) in addition to an antioxidant.

A PRokEEP low protein adsorption tube (manufactured by Watson) (capacity: 1.5 ml, indicated by "W" in FIG. 1) was also evaluated for comparison. This tube is formed using a low protein/peptide adsorption resin, and can withstand a centrifugal force of 20,000×G (physiological saline solution (1 ml), 15 min).

The proteomic analysis was performed as follows.

### Steps

(1) BSA (100 ng or 40 ng) was subjected to SDS-PAGE (6 lanes each, 12 bands in total) (CBB staining).
(2) The BSA band was cut, and put in each sample container (3×4=12 sample containers), and the gel strip was washed (100 mM NH₄HCO₃, 50% ACN (acetonitrile), 37°C, 20 min×2).
(3) Reduction was effected using DTT (60°C, 1 hour).
(4) Alkylation was effected using iodeacetamide (25°C, 1 hour).
(5) Enzymatic digestion was effected using trypsin (37°C, about 15 hours).
(6) Digested peptides were extracted (0.1% TFA/50% ACN (50 µl), 37°C, 20 min×2), and the extract was put in another sample container.
(7) A half quantity of the solution was respectively dispensed in identical sample containers (2×2×6=24 sample containers).
(8) One of each pair was concentrated to about 40 µl. The other of each pair was dried, and dissolved in 50% ACN/0.1% TFA (50 µl), followed by the addition of 35 µl of 0.1 % TFA.
(9) After performing a centrifugal operation for 7 minutes at a centrifugal force of 17400g, the supernatant liquid was analyzed by nanoLC, and the eluate was spotted onto a MALDI sample plate.
(10) After performing MALDI-MS/MS measurement, a MASCOT database search was performed.

### Equipment

Mass spectrometer (Maldi-TOF/TOF): 4700 Proteomics Analyzer (Applied Biosystems, Framingham, MA)
HPLC: Prominence nano (Shimadzu Corporation)
HPLC analysis conditions
System: Prominence nano (Shimadzu Corporation)
Concentrating column: Monolith (I.D.: 200 µm, length: 100 mm) (Kyoto Monotech)
Concentrating column washing solution: 0.1% TEA/H₂O, flow rate: 50.01/min
Separation column: Monolith (LD.: 100 µm, length: 250 mm) (Kyoto Monotech)
Eluent A (A%): 0.1 % TFA/5% MeCN, eluent B (B%): 0.1 % TFA/90% MeCN
Flow rate: 1.0 µl/min, detection: UV 210 nm, sample injection volume: 10 µl

FIG. 1 shows the digested peptide fragment recovery results (in-gel digestion of BSA).

In FIG. 1, "conc." indicates the concentrated sample, and "dried" indicates the dried sample (see the step (8)). "coverage" indicates the ratio of the total number of amino acids of matched peptides to the total number of amino acids of proteins (i.e., the coverage of the amino acid sequence in the Mascot search results).

"matched.pep." indicates the number of matched peptides.

It was confirmed that the sample containers RA, RB, and RC produced using the homopropylene resin that does not contain an additional additive other than the antioxidant and the neutralizer had a high digested peptide fragment recovery effect (i.e., exhibited an excellent peptide adsorption prevention capability). The sample container RB had the most excellent effect in the group "25 ng-conc.", and the sample container RC had the most excellent effect in the groups "25 ng-dried" and "10 ng-conc.".

The sample containers RA, RB, and RC exhibited an excellent peptide adsorption prevention capability as compared with the commercially available product W. The above effect was observed when the content of the hydrogenated derivative in the resin composition was about 10% or more. The content of the hydrogenated derivative is preferably 80% or less from the viewpoint of moldability. The content of the hydrogenated derivative is more preferably 10 to 60%.

The sample containers RA, RB, and RC and the commercially available product W were subjected to an eluate comparative analysis test (simple elution test). 300 µl of a mixed solvent of water, 50% ACN, and 0.1 % TFA was put in the sample container (twelve sample containers in total), and the sample container was shaken at 40°C for 40 minutes.

The sample in the first group was concentrated to about 40 µl. The sample in the second group was dried, and then dissolved in 40 µl of 0.1 % TFA.

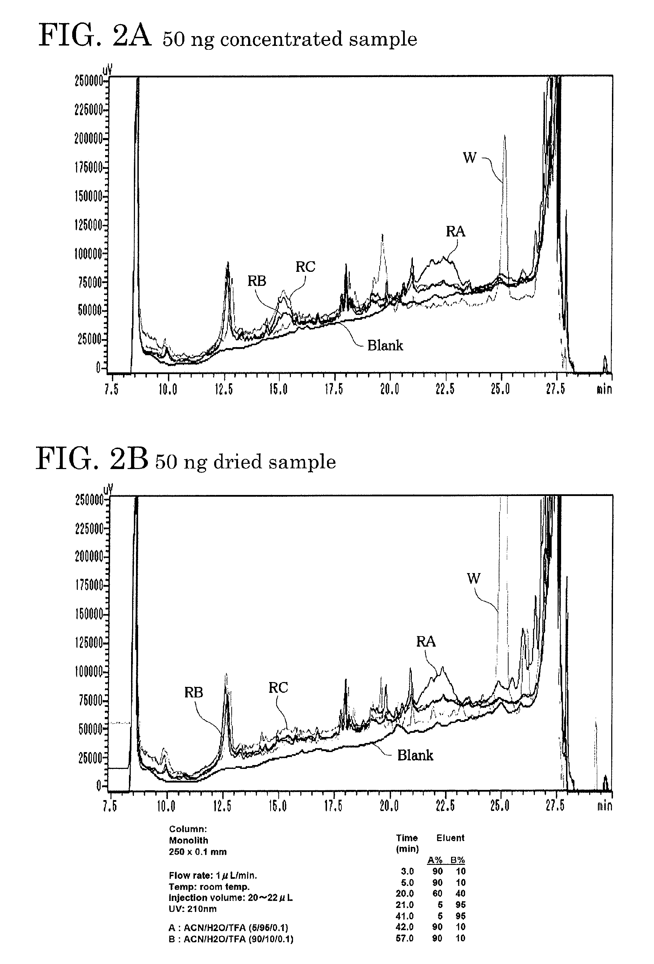

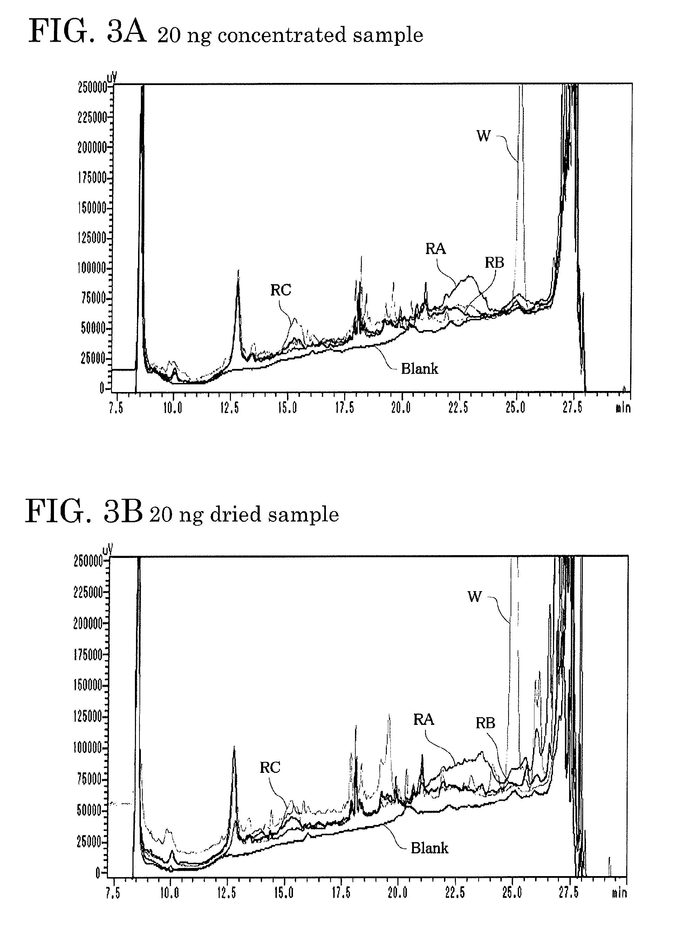

20 µl of each sample was analyzed by LC. FIGS. 2 and 3 show the test results. Results for 50 ng concentrated sample and dried sample

When using the sample container RA, UV absorption was observed for the concentrated sample and the dried sample at about 22 minutes. When analyzing the concentrated sample, the highest UV absorption was observed when using the sample container RB, and the second highest UV absorption was observed when using the sample container RA. These absorptions were not significant when analyzing the dried sample. When using the sample container RA, high UV absorption was observed for the dried sample in the latter part of the chromatogram as compared with the concentrated sample. When using the commercially available product W, high absorption was observed for the dried sample at about 25 minutes as compared with the concentrated sample.

### Results for 20 ng concentrated sample and dried sample

When using the sample container RA, UV absorption was observed for the concentrated sample and the dried sample at about 22 minutes. The highest UV absorption was observed for the concentrated sample and the dried sample at about 12 minutes when using the sample container RA, and the second highest UV absorption was observed when using the sample container RB. When using the sample container RA, high UV absorption was observed for the dried sample in the latter part of the chromatogram as compared with the concentrated sample. When using the commercially available product W, high absorption was observed for the dried sample at about 25 minutes as compared with the concentrated sample.

The sample containers RA, RB, and RC showed almost equal results in the adsorption test. In the elution test, UV absorption observed in the latter part of the chromatogram when using the sample container RA was higher than that observed when using the sample containers RB and RC. Therefore, the sample containers RB and RC showed better overall results.

### Comparative test

A comparative test was performed using a sample container RD.

The sample container RD was produced using a material obtained by mixing (kneading) a random polypropylene resin "J-3021GR" (manufactured by Idemitsu Kosan Co., Ltd.) and a hydrogenated polystyrene-vinylpolyisoprene-polystyrene block copolymer "Hybrar 7311" (manufactured by Kuraray Ltd.) in a ratio of 50:50. The CAS number of the random polypropylene resin "J-3021GR" is 9003-07-0 and 9010-79-1. The random polypropylene resin "J-3021GR" contains a UV absorber, an antistatic agent, a nucleating agent, and a slipping agent.

The random polypropylene resin "J-3021GR" has low chemical resistance.

BSA (10 ng or 50 ng) was subjected to SDS-PAGE (silver staining). The BSA band was cut, and subjected to denaturation, reduction, and alkylation. Enzymatic digestion was then effected using trypsin (about 17 hours). Digested peptides were extracted, and the extract was concentrated (20 µl) or dried.

The dried sample was dissolved in 0.1% TFA/H₂O (20 µl).

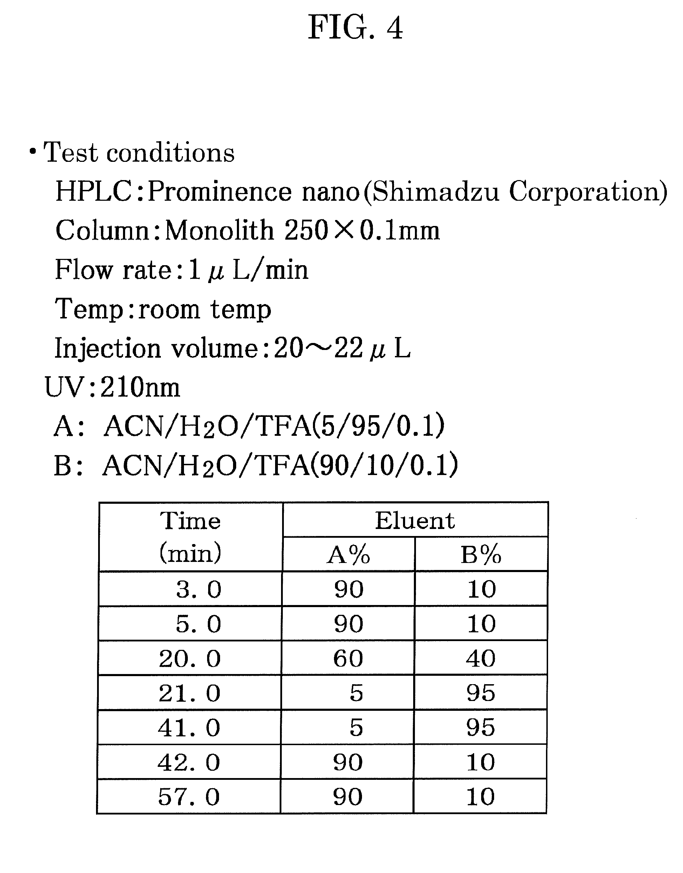

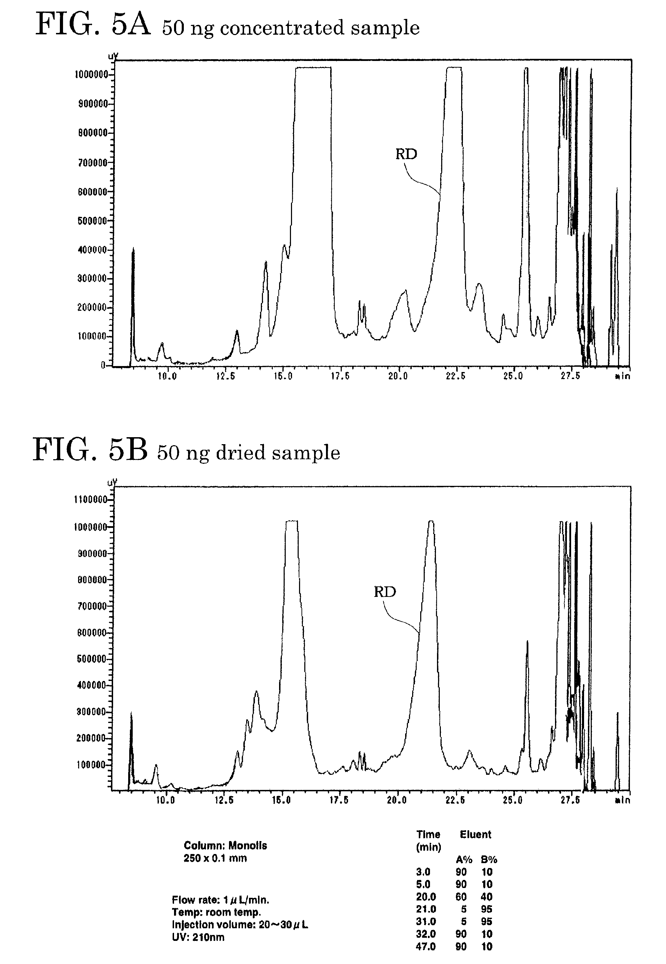

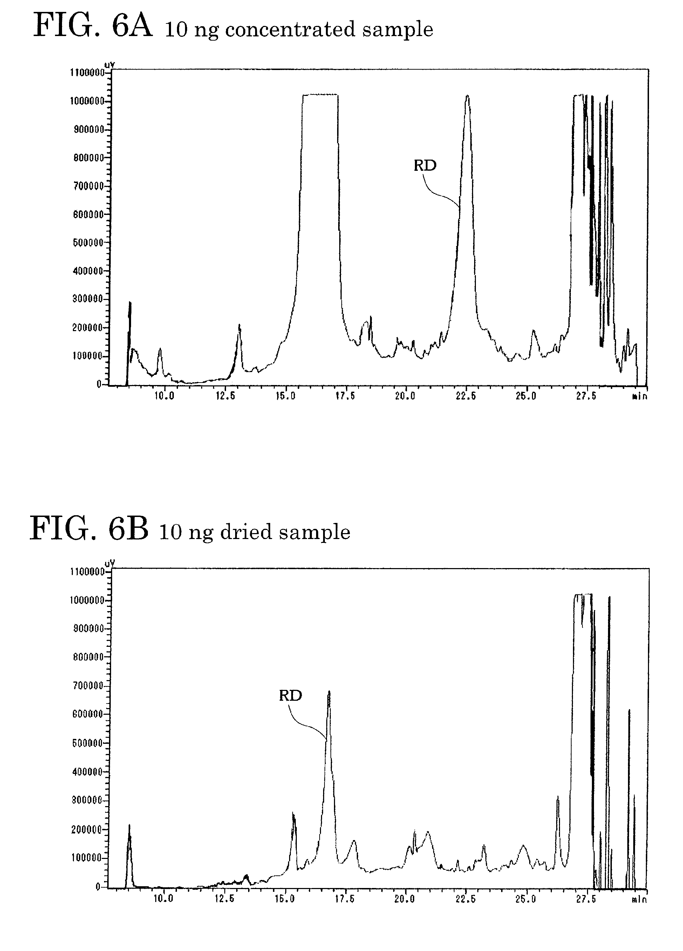

FIG. 4 shows the test conditions, and FIGS. 5 and 6 show the test results (LC analysis).

The peptides adsorbed on the column were sequentially eluted while changing the mixing ratio of the A% liquid and the B% liquid (i.e., changing the hydrophilicity and the hydrophobicity of the eluent) with the passage of time.

The amount of peptides detected normally becomes the largest when the A% liquid is 30% (retention time: about 15 minutes, hereinafter referred to as "first part"), and becomes the next largest when the A% liquid is 50% (retention time: about 20 minutes, hereinafter referred to as "second part").

A very high absorption peak was observed in the first part and the second part of the UV spectrum.

Since the eluate to which the absorption peak is attributed hinders LC UV detection and mass spectrometry during proteomic analysis, it is desirable to use a sample container that does not produce an eluate as much as possible. It is considered that the above high absorption peak was observed because the compounds (e.g., antistatic agent and plasticizer) added to the resin material were eluted from the inner wall of the sample container in each proteomic analysis step (particularly the step (6).

### Mechanical strength of sample container

Japanese Patent Application No. 2003-188064 (JP-A-2005-023148) applied for by the applicant of the present application discloses that a decrease in melting point does not occur, and a decrease in mechanical strength is small when the polymer block Y is miscible with the polypropylene-based resin.

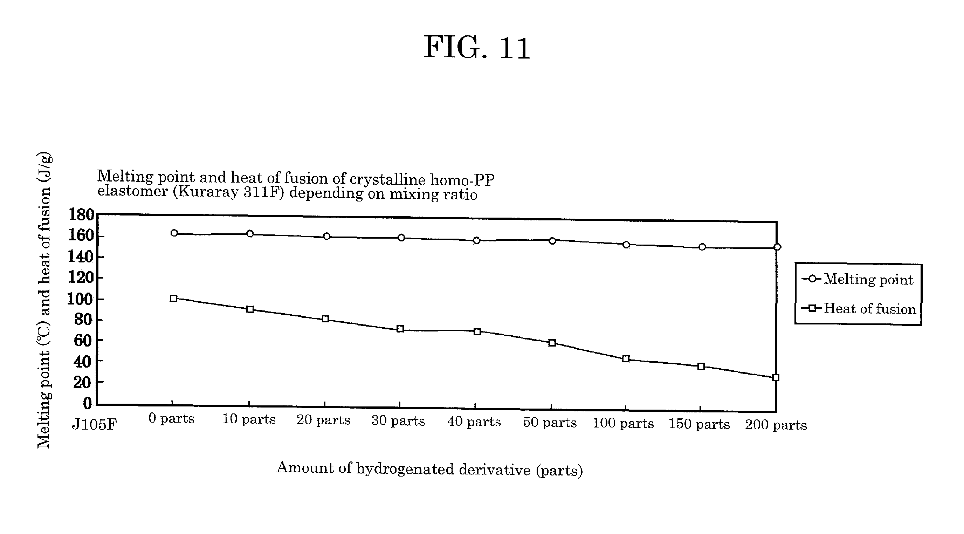

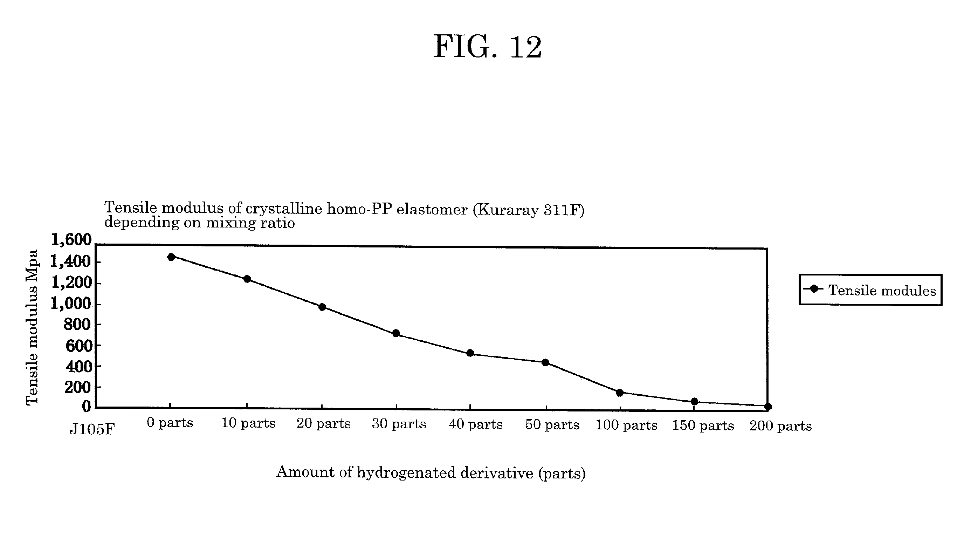

As shown in FIGS. 11 and 12, a high melting point is maintained regardless of the presence or absence of the hydrogenated derivative. The heat of fusion decreases as the mixing ratio of the hydrogenated derivative increases. However, a certain level of heat resistance is maintained even when the mixing ratio is 50:50.

The tensile modulus decreases as the mixing ratio of the hydrogenated derivative increases. However, the tensile modulus is 1500 MPa when the mixing ratio is 100%, and is 200 MPa when the mixing ratio is 50:50. These values are sufficiently larger than the value (100 MPa or more) required for an injection-molded product.

Since the tensile modulus of the homopolypropylene resin "MA04A" is 2050 MPa, it is considered that the tensile modulus of the resulting product is sufficiently higher than 200 MPa.

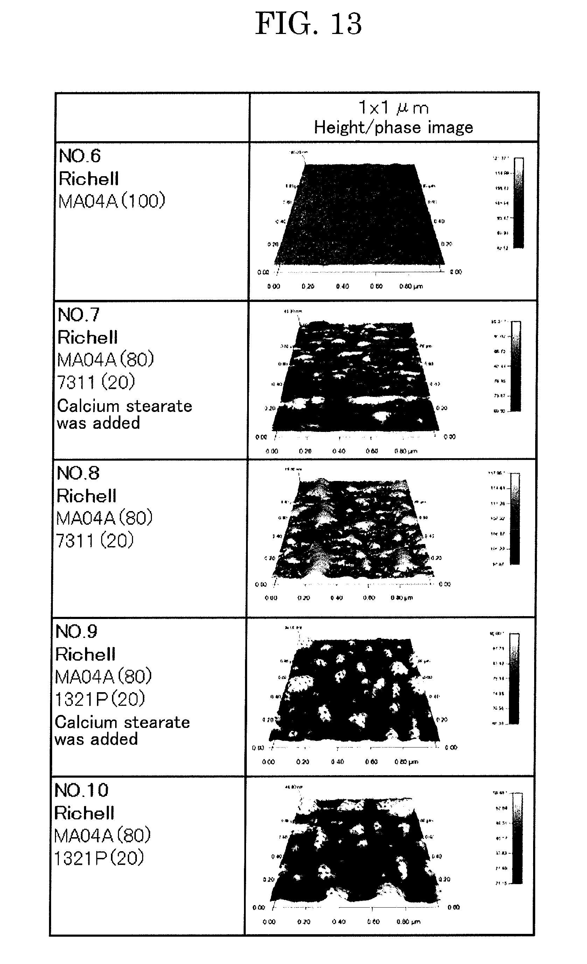

FIG. 13 shows an atomic force micrograph of the inner surface of the main body of a sample container (see FIG. 9) produced by injection-molding a resin composition containing 80% of the polypropylene-based resin "MA04A" and 20% of the hydrogenated derivative "7311" or "1321P", or a resin composition containing 80% of the polypropylene-based resin "MA04A", 20% of the hydrogenated derivative "7311" or "1321P", and 0.2% of calcium stearate (anti-blocking agent).

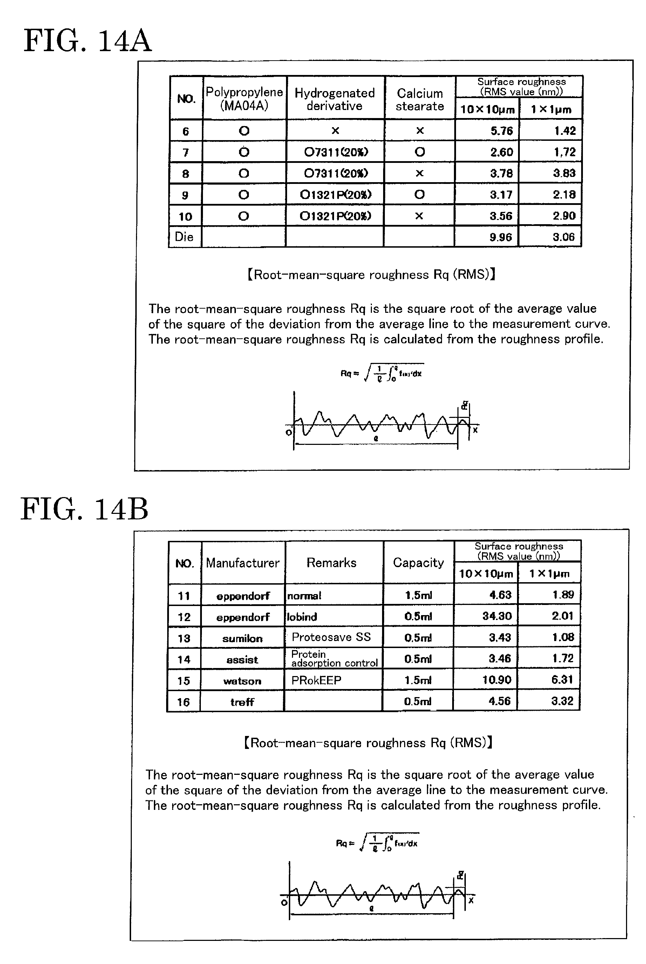

FIG. 14(a) shows the measurement results for the root-mean-square surface roughness Rq (corresponding to FIG. 13).

FIG. 14(b) shows the measurement results for the root-mean-square surface roughness of a commercially available product.

Note that "10×10 µm" and "1 × 1 µm" in FIGS. 14(a) and 14(b) indicate the size of the image area.

The atomic force micrograph (AFM) shows that the hydrogenated derivative is dispersed in the polypropylene-based resin phase in the shape of islands.

The resin molded products (No. 7 to No. 10) according to the embodiments of the invention had a surface roughness of 5 nm or less.

The commercially available product "Eppendorf (LoBind)" and the commercially available product manufactured by Watson had a surface roughness of more than 10 nm.

The sample containers were subjected to a peptide adsorption test, an elution test, and a liquid removal test.

Abbreviations for the test samples are shown below.
(1) R08 (inventive product corresponding to No. 8 in FIG. 13)
   The following test samples are commercially available products.
(2) SPSS ("SUMILON Proteosave SS" manufactured by Sumitomo Bakelite Co., Ltd.)
(3) EPLB (Eppendorf Protein LoBind Tube (0.5 ml) manufactured by Eppendorf)
(4) AMPC (Assist protein adsorption control sampling tube (0.5 ml) manufactured by Assist)
(5) WPK (PRokEEP manufactured by Watson)
(6) E3810 (Eppendorf Sampling Tube 3810 manufactured by Eppendorf)
(7) TREF (Treff EasiFit Microtube (0.5 ml) manufactured by Treff)

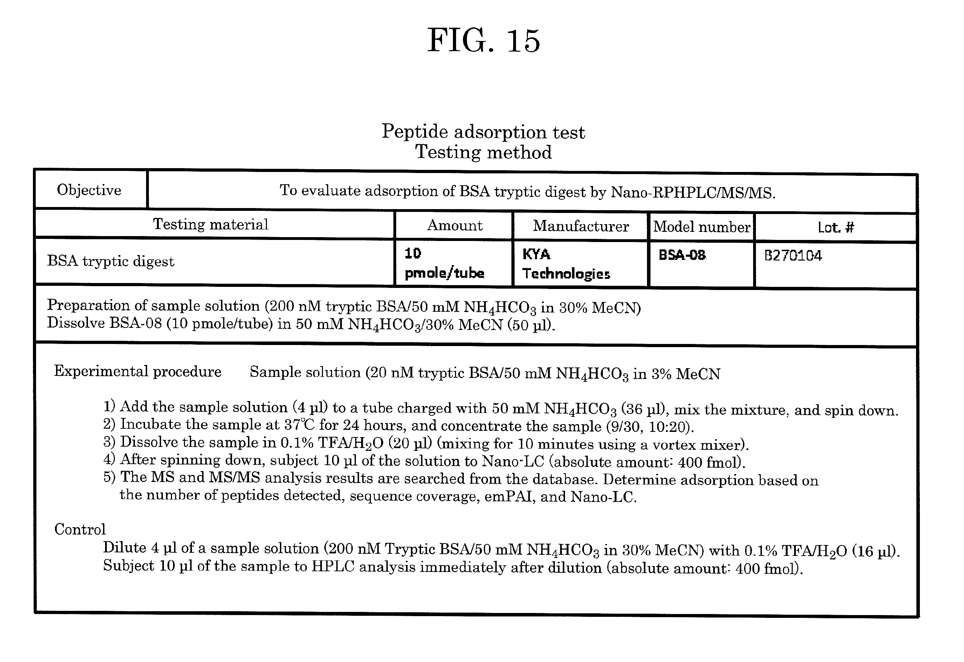

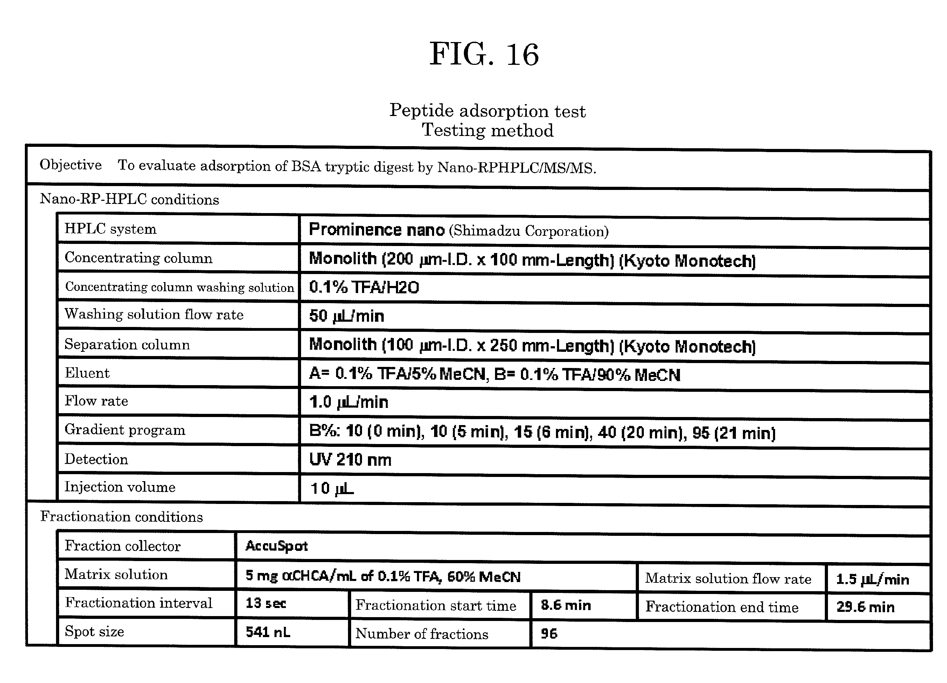

The proteomic analytical process and the HPLC conditions are shown in FIGS. 15 and 16.

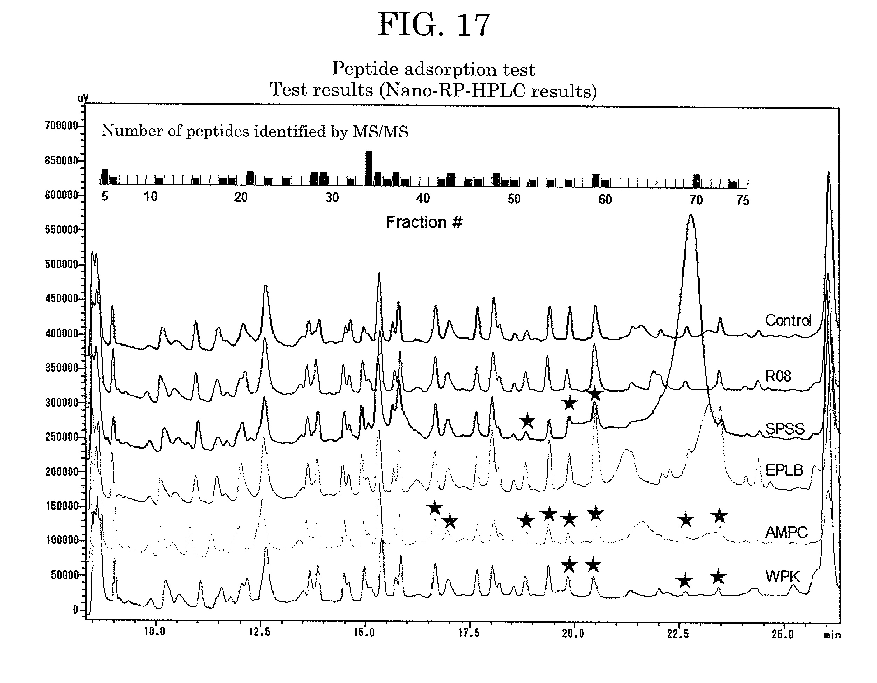

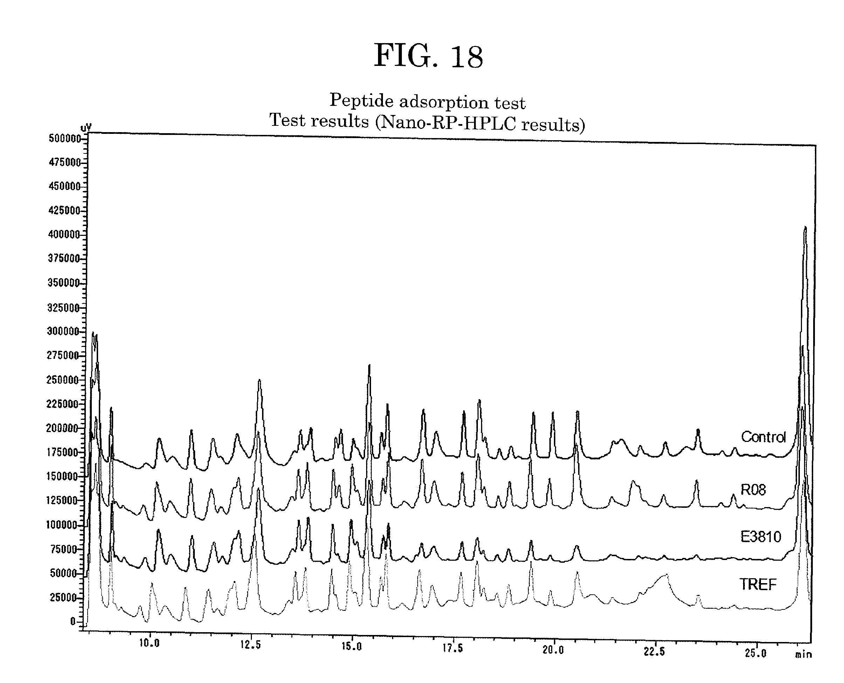

FIGS. 17 and 18 show the number of peptides identified by MS/MS and the UV spectrum of each sample.

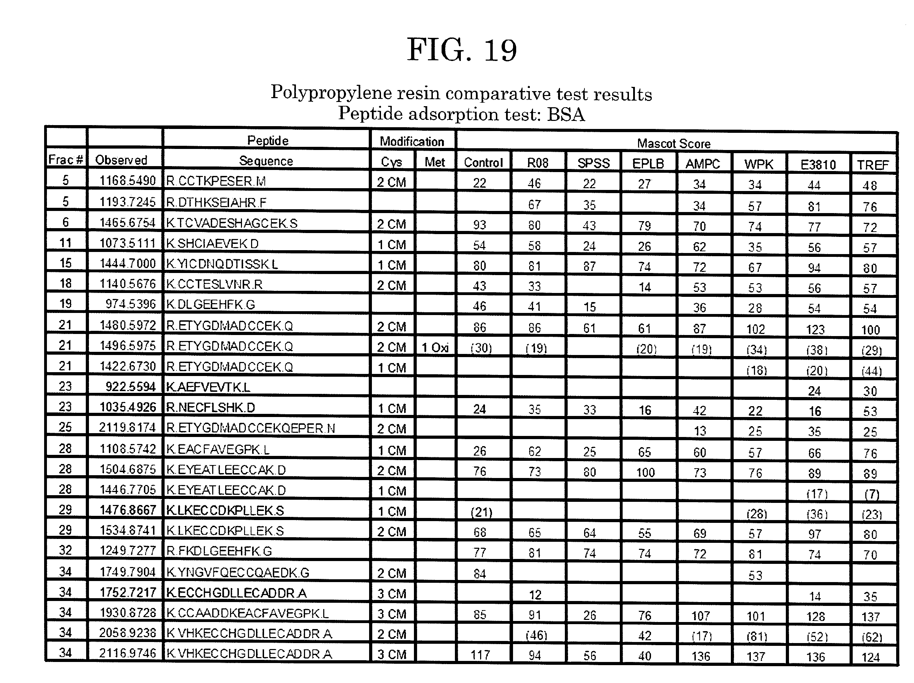

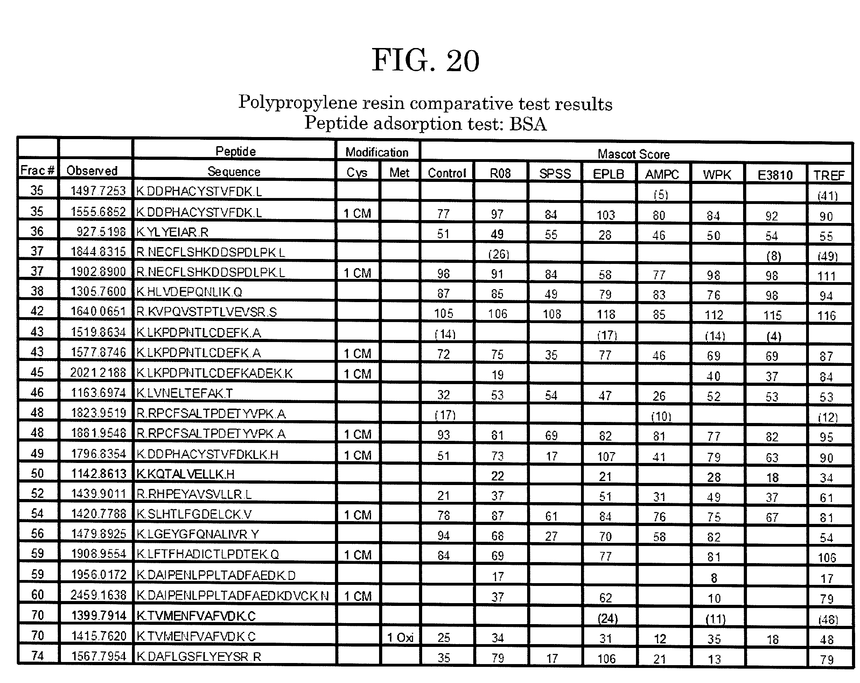

FIGS. 19 and 20 show the Mascot score of each fraction identified by MS/MS. In FIGS. 17 and 18, each star sign indicates that the peak height was lower than the number of peptides obtained from the control (i.e., peptides were adsorbed on the sample container, and were not detected from the recovered liquid).

It was thus confirmed that the test sample "R08" (inventive product) showed suppressed peptide adsorption as compared with the commercially available products.

Note that a large total peak area in the analytical chart shown in FIGS. 17 and 18 indicates an excellent capability to prevent adsorption of proteins, polypeptides, peptides, and the like.

The elution test results are described below.

Dimethysulfoxide (DMSO), acetone, and 0.1 % TFA (in 50% MeCN) were used as the eluent in the elution test.

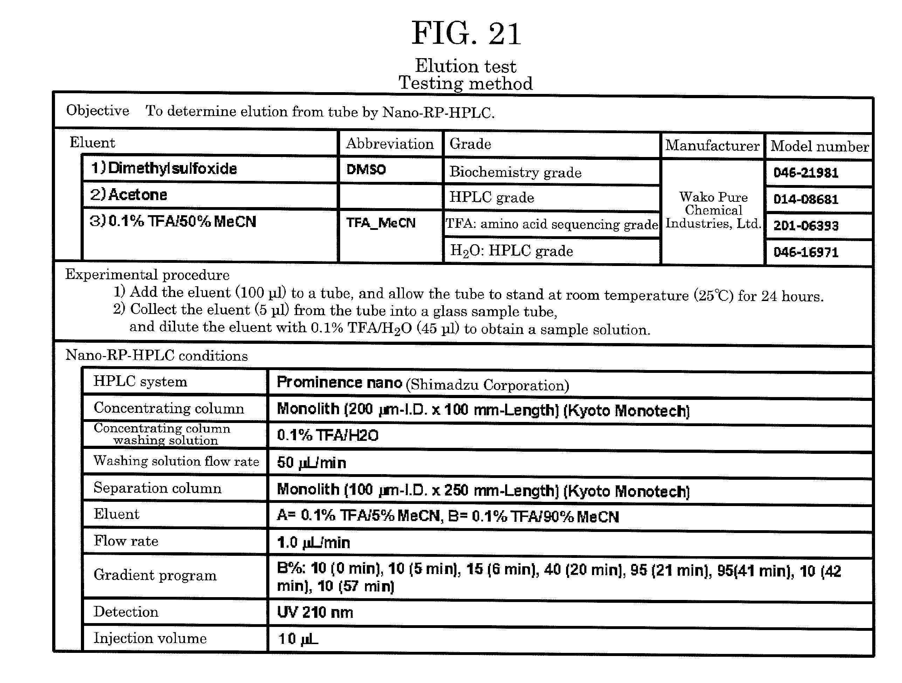

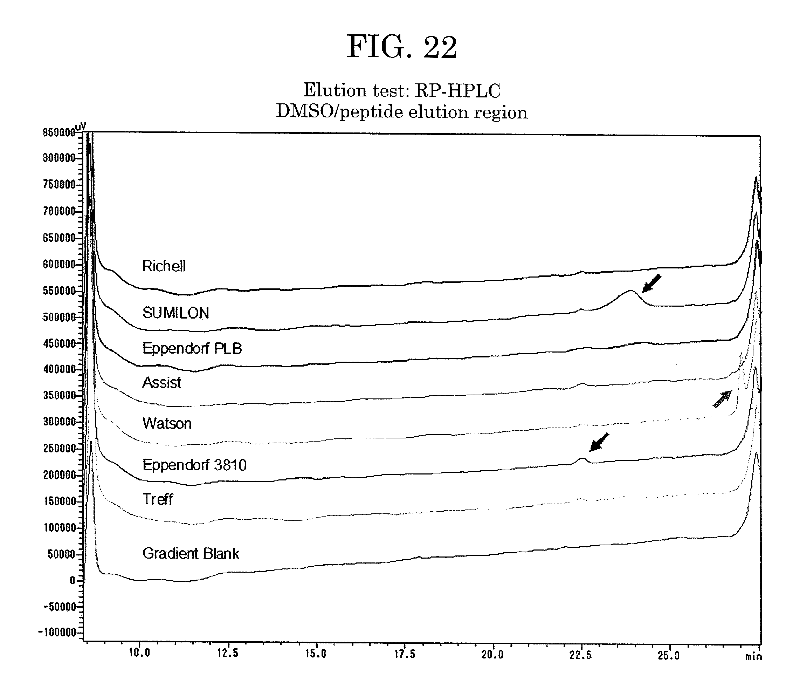

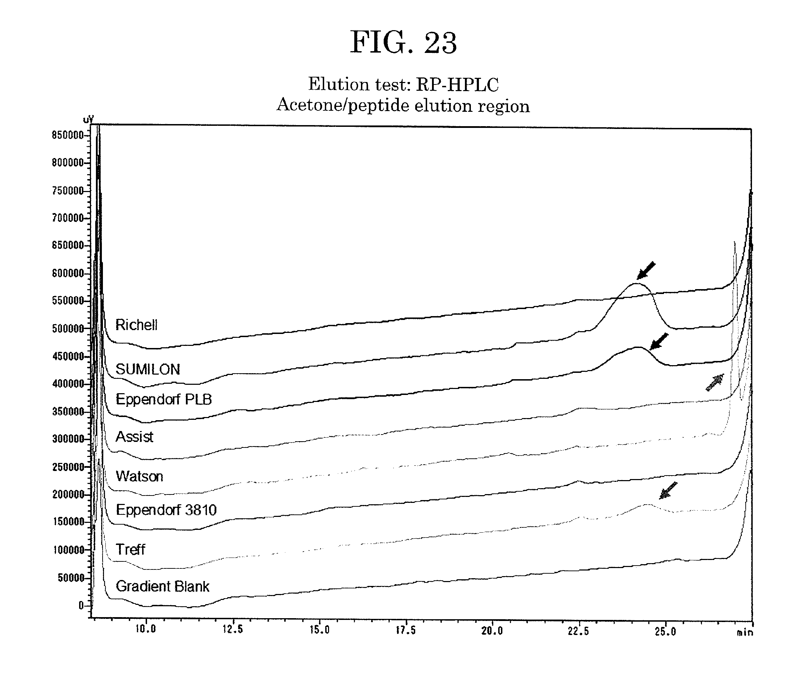

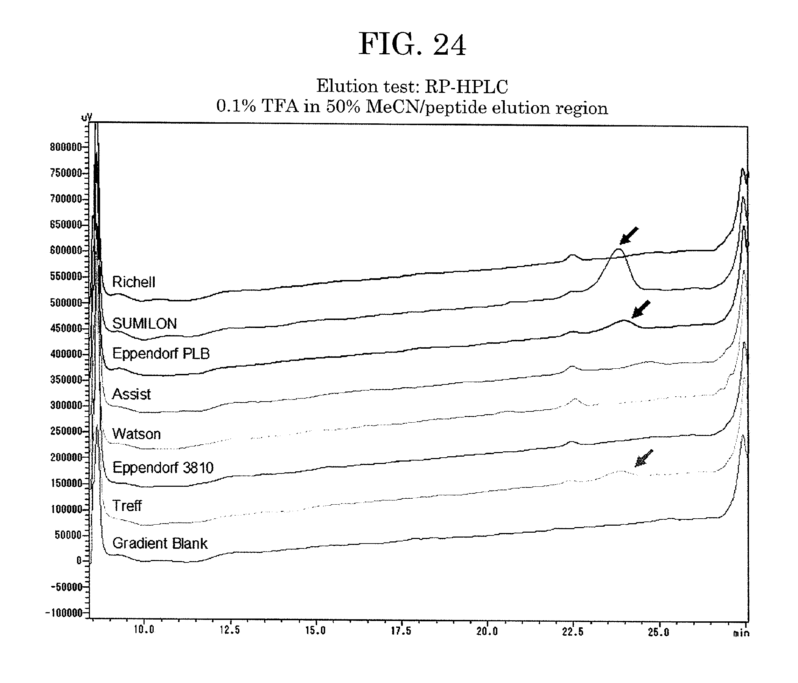

FIG. 21 shows the elution test conditions, and FIGS. 22 to 24 show the HPLC detection chart. In the HPLC detection chart, an eluate is indicated by an arrow.

An eluate was not detected when using the test sample "Richell" (inventive product) regardless of the type of eluent.

The liquid removal test results are described below.

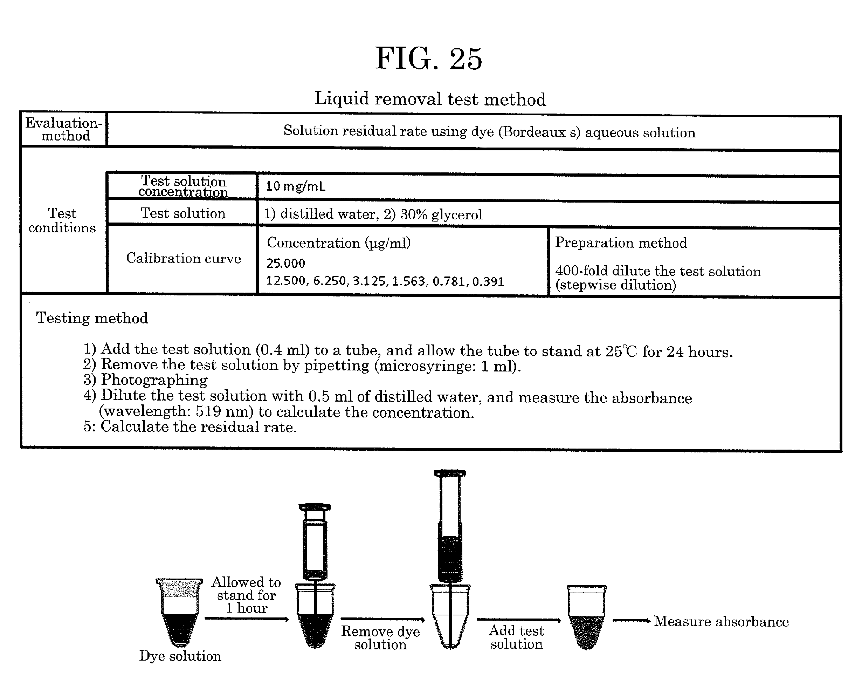

As shown in FIG. 25, a dye solution was injected into the sample container. After allowing the sample container to stand for 1 hour, the dye solution was removed, and the presence or absence and the concentration of the dye that remained in the sample container were measured.

Distilled water and a 30% glycerol aqueous solution were used as the solvent.

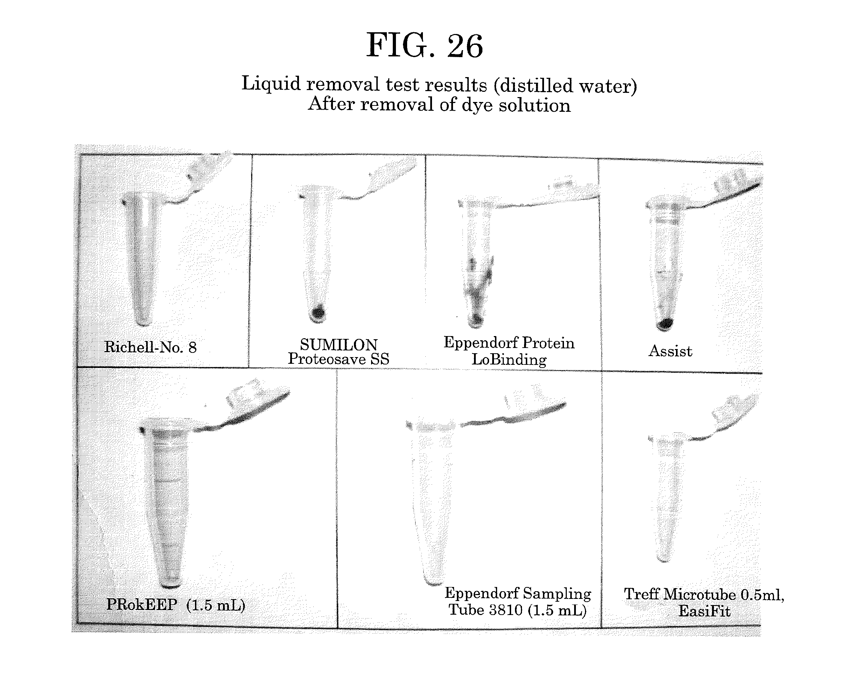

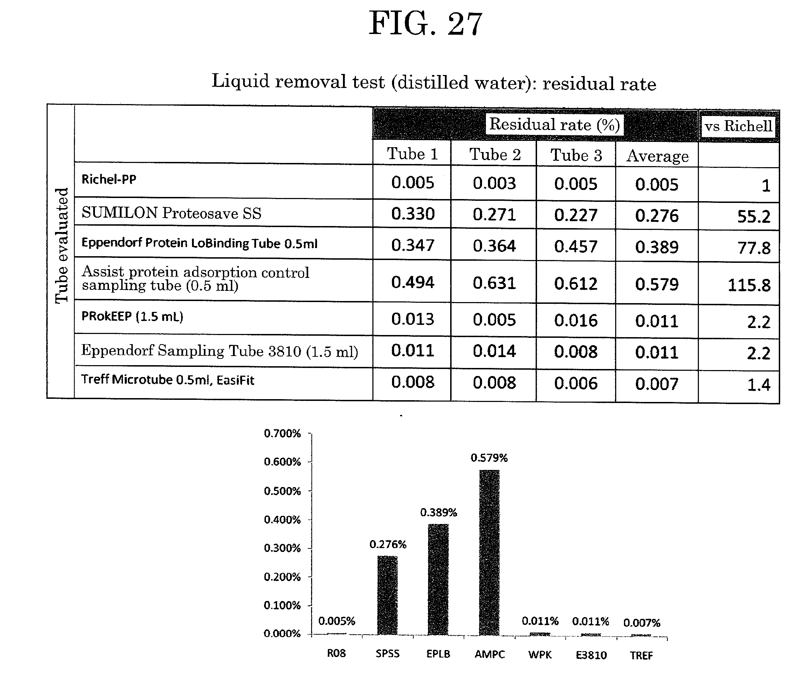

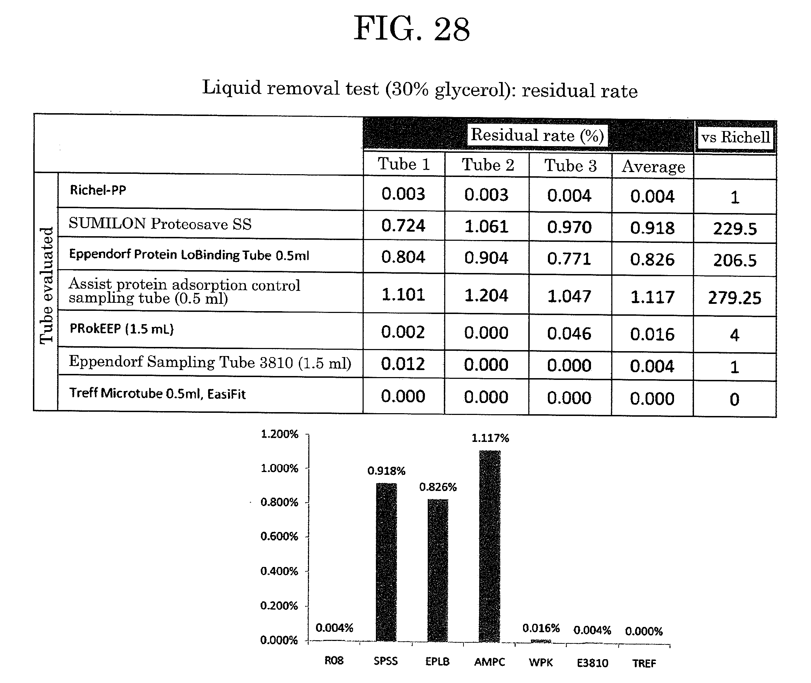

FIG. 26 shows the test results (photograph), and FIGS. 27 and 28 show the dye residual rate.

In the photograph shown in FIG. 26, a colored area at the bottom of the sample container indicates that the dye remained.

The dye residual rate was very low when using the test sample "R08" (inventive product). Therefore, the test sample "R08" may effectively be used for proteomic analysis and the like.

### INDUSTRIAL APPLICABILITY

The invention relates to a resin composition that exhibits excellent moldability and excellent mechanical strength, and may suitably be used to produce a resin product that suppresses elution from the inner wall thereof. In particular, the resin composition may be used as a medical or biochemical resin composition that is required to prevent adsorption of proteins, polypeptides, and peptides, and exhibit biocompatibility, antithrombogenicity, safety (elution resistance), and mechanical strength.

Specific examples of medical products produced by molding the resin composition using various molding methods (e.g., compression molding, injection molding, or extrusion molding) include an artificial organ, an artificial vessel, a connector, a catheter, a stent, a tube, a blood circuit, a blood storage container, a dialyzer potting material, an oxygenator housing, a permeable membrane, a glass syringe, a syringe needle, a suture, a dialyzer, a cannula, a shunt, and the like.

## Claims

1. A medical or biochemical resin composition comprising a polypropylene-based resin, and a hydrogenated derivative of a block copolymer shown by X-Y (wherein X represents a polymer block immiscible with the polypropylene-based resin, and Y represents an elastomeric polymer block of a conjugated diene), the polypropylene-based resin having a content of an antioxidant of 1 mass% or less and a content of an additive of 0.5 mass% or less, the additive being used to disperse the antioxidant in the polypropylene-based resin.

2. A medical or biochemical resin composition comprising a polypropylene-based resin, and a hydrogenated derivative of a block copolymer shown by X-Y (wherein X represents a polymer block immiscible with the polypropylene-based resin, and Y represents an elastomeric polymer block of a conjugated diene), the resin composition having a content of an antioxidant of 1 mass% or less, and having a total content of additional additives of 0.5 mass% or less.

3. The medical or biochemical resin composition as defined in claim 1 or 2, the polypropylene -based resin being a homopolypropylene resin.

4. The medical or biochemical resin composition as defined in any one of claims 1 to 3, the polymer block X of the hydrogenated derivative being polystyrene, and the polymer block Y before hydrogenation being 1,2-polyisoprene, 3,4-polyisoprene, and/or 1,4-polyisoprene.

5. The medical or biochemical resin composition as defined in any one of claims 1 to 3, the polymer block X of the hydrogenated derivative being polystyrene, and the polymer block Y before hydrogenation being 1,2-polybutadiene and/or 1,4-polybutadiene.

6. The medical or biochemical resin composition as defined in any one of claims 1 to 5, the resin composition having a content of the hydrogenated derivative of 10 to 60 mass%.

7. A medical or biochemical resin molded product obtained by injection-molding the medical or biochemical resin composition as defined in any one of claims 1 to 6, the resin molded product having a root-mean-square surface roughness of 10 nm or less.

8. The medical or biochemical resin molded product as defined in claim 7, the resin molded product having a tensile modulus of 100 MPa or more.
